# EUROPEAN PATENT APPLICATION

(11) **EP 1 674 058 A1**
(43) Date of publication of application: **28.06.2006**
(21) Application number: 04030645.8
(22) Date of filing: 23.12.2004
(51) Int. Cl.: A61F 11/06

(54) **Method and system for providing active hearing protection**

(71) Applicant: PHONAK AG, 8712 Stäfa (CH)
(72) Inventor: Haussmann, Mathias, 8005 Zürich (CH)
(74) Representative: Schwan - Schwan - Schorer

(57) **Abstract**

The invention relates to a method for providing hearing protection to a person, comprising: providing each of the person's ears with a hearing protection device (10, 12) in such a manner that an acoustic attenuation of at least 10 dB averaged over the audible frequency range is achieved; converting, by said hearing protection devices, ambient sound into input audio signals; assigning, by analyzing phases of said input audio signals, angular directions to said input signals; permanently determining noise directions (32, 42, 44, 46, 48) by searching, by analyzing levels of said input audio signals, over all angular directions around said person for angular directions having a noise source (30, 34, 36, 38, 40) from which noise impinges on said hearing protection devices, wherein an angular direction is judged to be a noise direction if input audio signals from that direction fulfil a predefined input sound level condition; processing, in order to produce processed audio signals, said input audio signals in such a manner that the levels of processed audio signals resulting from input audio signals from said noise direction(s) are reduced, relative to processed audio signals resulting from input audio signals from other angular directions, in order to comply with a predefined output sound level condition; and converting said processed audio signals into sound for being received by said person's ears.

## Description

The invention relates to a method for providing hearing protection to a person and to a hearing protection system.

A large part of the population is exposed to hazardous noise from time to time. This can be at work, whilst traveling, during leisure activities or at home. The exposure can lead to permanent hearing loss, distract people's attention from other hazards or simply cause stress. In order to prevent both accidents and permanent hearing damage, hearing protection devices (HPDs) have been provided in many styles and over many years. It started with the earmuff which is still very relevant and addresses very noisy environments (e.g. airports, construction, shooting) or complex working/communication situations (e.g. fighter pilots). Over the years development of biocompatible soft materials has enabled soft earplugs in different styles and colors as well as recent development of "one fits many" standard semi-soft earplugs in silicon-rubber type materials. For severe situations even the combination of an earmuff and an "in-the-ear" HPD is required to achieve desired attenuation. The physical limitation of hearing protection based on ear worn devices is defined where bone-conduction (body acoustics) becomes dominant at around 40 dB attenuation.

A common disadvantage of the above mentioned HPD styles is wearing discomfort. In case of the earmuffs, they are large which creates difficulties in combination with other head worn gear and they "close off' the ear too much for most applications. The in-the-ear styles mentioned are devices made to fit "the average" ear in one way or the other. Either the fit is provided by softness of the material which leads to undefined device insertion and undefined attenuation, or the fit is provided by standard shaped structures intended to block off the ear canal. In both cases the flat distribution of the individual shape of the outer ear and the ear canal leads to bad fit, pressure points in the ear and undefined positioning of the device.

To address this wearing comfort issue, in-the-ear hearing aid technology has been applied for making customized ear molds with passive acoustical filters. These are long lasting devices with good wearing comfort. However, this customization process is traditionally a very manual process creating varying results over time, low reproducibility and the quality is very operator skill dependent.

Customized earplugs are earplugs comprising a hard shell which has an outer surface individually shaped according to the measured inner shape of the user's outer ear and ear canal. Such earplugs presently are primarily used for housing hearing aids. The inner shape of the user's outer ear and ear canal may be measured, for example, by direct laser scanning or by forming an impression. The customized hard shell may be produced by an additive process, such as layer-by-layer laser sintering of a powder material. Customized earplugs are described, for example, in WO 02/071794 A1, US 2003/0133583 A1 or US 6,533,062 B1.

On the other hand, soft earplugs are widely used, in particular also as hearing protection devices. A soft earplug has an outer surface with a standardized shape is made of a relatively soft material so that the outer surface of the earplug is capable of adapting its shape to the individual inner shape of the user's outer ear and ear canal.

It is commonly known to design hearing protection devices as so-called active hearing protection devices wherein each device is provided with an outer microphone for converting ambient sound into input audio signals, a signal processing unit for processing the input audio signals into output audio signals, and an acoustic output transducer, i.e. speaker or receiver, which converts the audio output signals into sound perceivable by the user when wearing the hearing protection device. Thereby the hearing protection device is provided with a communication function enabling the user to perceive ambient sound signals in a controlled manner even when wearing the hearing protection device in a noisy environment. In order to provide for a selective communication function, i.e. in order to enable the user to perceive, for example, speech while suppressing undesired noise, it is known to provide the audio signal processing unit with the capability of assessing the sound picture and adapting filter and gain settings to the noise level dynamically.

An example of such a system is described in US 4,677,678 which relates to hearing protection devices with an active processing and adaptive circuitry (AGC), with one AGC taking care of the sound in both ears where the hearing protection devices are electrically connected. US 2001/0046304 A1 relates to a headset device for providing external audio signals to the user's ear having a hear-through function for selected audio signals, which is achieved by analyzing the ambient sound for predetermined stored sound signals and by selectively amplifying the ambient sound in this mode over the external audio signals to be provided to the user's ear by the headset. WO 02/17837 A1 relates to an active hearing protection device which comprises, in addition to the outer microphone, an inner microphone picking up sound at the distal end of the hearing protection device, wherein desired sound is selectively filtered and amplified based on input audio signal analysis.

For hearing aid systems it is well known to provide for a beam forming functionality by providing the hearing aid system with two spaced apart microphones whereby spatial resolution of the sensed ambient sound can be achieved by analyzing the phase difference between the input audio signals provided by the two microphones. Examples of such beam forming hearing aid systems are given in US 5,473,701, EP 1 005 783 B1, US 6,522,756, EP 1 269 576 A1, EP 1 391 138 A2 and EP 1 320 281 A2. Usually the beam forming technique in hearing aids is used for determining the angular direction of a source of speech relative to the user of the hearing aid system in order to selectively amplify speech in this angular direction over background noise. In other words, usually the amplification is maximized in the direction in which presently an acoustic signal source is detected.

EP 1 320 81 A1 discloses a hearing aid system wherein alternatively, if the acoustic signal source is a noise source, the amplification in this noise source direction is minimized in order to cancel this noise source from the sound signals provided to the hearing aid user by the acoustic output transducer.

Usually the angular tracking in beam forming hearing aid systems is carried out in a plane around the user's head. However, WO 00/68703 discloses a hearing aid system which enables three-dimensional localizing of sources of acoustic signals, wherein, if the localized acoustic signal source has been identified as a noise source, the amplification in this direction is reduced.

It is an object of the invention to provide for a hearing protection method and a hearing protection system which enable the user to perceive selected acoustic signals even when wearing the hearing protection system, while at the same time the user is protected from excessive sound levels.

This object is achieved by a hearing protection method as defined in claim 1 and a hearing protection system as defined in claim 17.

This solution is beneficial in that, by permanently determining noise directions over all angular directions around the user and by reducing the levels of processed audio signals resulting from such detected noise directions relative to processed audio signals resulting from other angular directions, an active hearing protection function may be provided which, on the one hand, allows the user to perceive selected acoustic signals even when wearing the hearing protection device in a noisy environment, while, on the other hand, the user is safely protected from excessive ambient sound levels produced by noise sources.

Preferred embodiments of the invention are defined in the dependent claims.

In the following, examples of the invention will be illustrated by reference to the attached drawings.
- Fig. 1: shows a schematic representation of a hearing protection system according to the invention; and
- Figs. 2 to 4: show schematic top views of a user wearing a hearing protection system according to the invention in three different typical noise exposure situations.

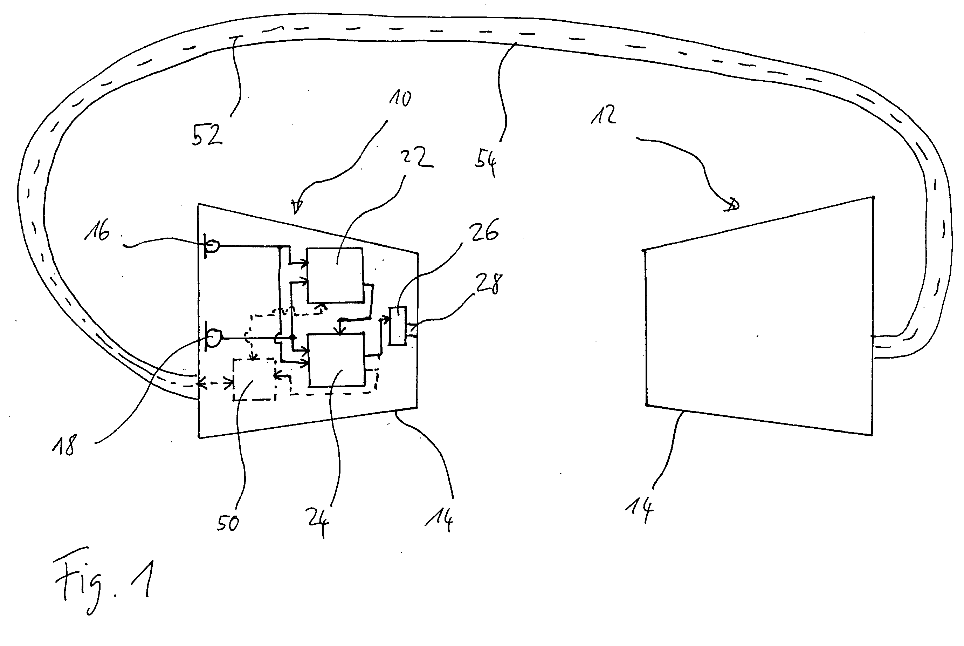

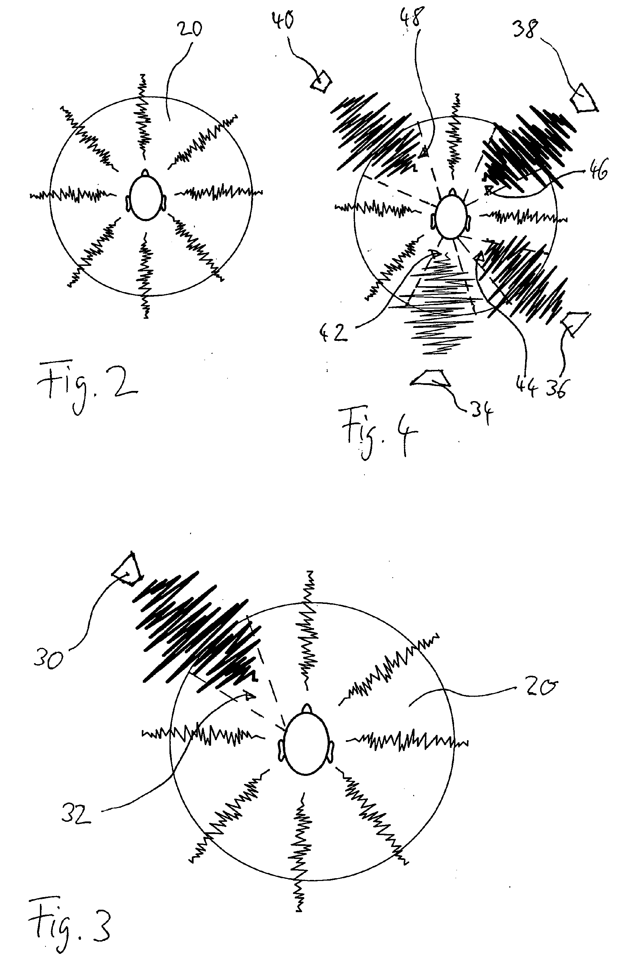

Fig. 1 is a schematic representation of a hearing protection system for a person comprising a hearing protection earplug 10 which is to be worn at least partly within a person's right ear canal and a hearing protection earplug 12 which is to be worn at least partly within the person's left ear canal. In a less preferred embodiment, the earplugs 10, 12 may be replaced by hearing protection devices which are to be worn at the person's right and left ear, respectively, such as an earmuff.

In general, the hearing protection devices according to the invention are adapted to provide for an acoustic attenuation of at least 10 dB averaged over the audible frequency range when worn by the user.

Each hearing protection earplug 10, 12 comprises a shell 14 which is adapted to be worn at least in part in a user's ear canal, i.e. at least a distal portion of the shell is to be inserted into the outer part of the user's ear canal, in order to provide for an acoustic attenuation of at least 10 dB averaged over the audible frequency range when the earplug is worn by the user in order to protect the user from excessive levels of ambient sound. The earplug may comprise an acoustic filter for adjusting the desired total acoustic attenuation or for adjusting the frequency dependent acoustic attenuation.

The shell preferably is a hard shell having an elasticity from shore D85 to D65 and preferably is made of polyamide. In order to achieve optimized fit of the shell within the user's outer ear and ear canal, the shell has an outer surface individually shaped according to the measured shape of the user's outer ear and ear canal, i.e. the shell has an individually customized outer shape. The shape of the user's outer ear and ear canal may be determined by direct three-dimensional scanning of the ear canal and the concha or by producing an impression of the ear canal and the concha which subsequently undergoes scanning. The scanning process may be carried out optically, preferably by laser scanning.

The digital data obtained by the scanning process is then used to create the hard shell by an additive or incremental layer-by-layer build up process. Such processes are also known as "rapid prototyping". A preferred additive build-up process is a layer-by-layer laser sintering process of powder material, preferably polyamide powder. Such processes are also known as "selective laser sintering" (SLS). The basic principle therein is the repeated deposition of a thin layer of material on a surface, with the desired sectional shape then being stabilized, i.e. hardened, by laser action. Other preferred additive layer-by-layer build-up processes are laser stereo-lithography or photo-polymerization. An overview regarding additive layer-by-layer build-up processes for producing customized shells for hearing aids can be found, for example, in US 2003/0133583 A1 or US 6,533,062 B1.

In the following, only the construction and functionality of the right earplug 10 will be discussed in detail. However, in a preferred embodiment the left earplug 12 will have the same construction with the same components and hence will provide for the same functionality.

The earplug 10 comprises two microphones 16 and 18 for converting ambient sound reaching the outer, i.e. proximal, end of the earplug 10 into corresponding input audio signals. The microphones 16 and 18 are spaced apart by a certain distance in order to enable an angular resolution of ambient sound signals by analyzing the phase differences between the input audio signals provided by the microphone 16 and the microphone 18, respectively. With two microphones 16 and 18 an angular resolution corresponding to the envelopes of cones which are coaxial to the (imaginary) axis connecting the two microphones and which have an angular aperture ϕ is achieved. The detected phase difference corresponds to a certain value of ϕ. Hence, by analyzing phases of the input audio signals from the microphones 16 and 18, angular directions ϕ corresponding to the envelope of a cone having an angular aperture ϕ can be assigned to the input audio signals. This general principle, when applied to a hearing aid, is described for example in EP 1 320 281 A1, WO 00/68703, EP 1 005 783 B1, US 6,522,756 B1, WO 00/33634, WO 01/60112, and EP 0 802 699 A2. In addition, a general description of this principle is found in US 5,473,701, while applications to mobile phones are described in US 5,400,409 and US 5,539,859.

According to the invention, the hearing protection system permanently searches for noise directions over all angular directions around the user, wherein a noise direction is an angular direction from which noise impinges on the hearing protection devices. In the case in which two microphones 16, 18 are employed, a noise direction would correspond to the envelope of a cone having a certain angular aperture (ϕ_{N}. In order to detect all present noise sources, all such cone envelopes would be searched by varying ϕ from 0 to 180 degrees. The noise directions are determined by analyzing levels of the input audio signals from the microphones 16 and 18 in an analyzer unit 22, wherein an angular direction is judged to be a noise direction if the input audio signals assigned to this angular direction by analyzing phases of the input audio signals fulfil a predefined input sound level condition.

In general, the invention relates to active hearing protection devices, i.e. hearing protection devices which do not only provide for an acoustic attenuation of ambient sound in order to protect the user's hearing, but in addition comprise means for electro-acoustically by-passing this acoustic attenuation function in order to provide the user with a communication function, e.g. in order to enable the user to perceive speech signals, even when wearing the hearing protection devices in a noisy environment. To this end, in the example of Fig. 1 each earplug 10, 12 is provided with an audio signal processing unit 24 for processing the input audio signals provided by the microphones 16, 18 into output audio signals which are converted into sound by an acoustic output transducer, i.e. speaker, 26, which sound is perceivable by the user's ears via a sound output opening 28 provided at the distal end of each earplug 10, 12.

In practice, the audio signal processing unit 24 and the analyzer unit 22 may be realized by a digital signal processor which includes a memory for storing programs and data. Consequently, the system in addition will comprise an analogue to digital converter between the microphones 16, 18 and the audio signal processing unit 24 / analyzer unit 22 and a digital to analogue converter between the audio signal processing unit 24 and the speaker 26 (these converters are not shown in Fig. 1). Further, the audio signal processing unit 24 and the analyzer unit 22 may be provided with an interface for temporarily connecting with a remote unit via a wireless or wired data connection (these components are not shown in Fig.1) for uploading programs and data into the audio signal processing unit 24 and the analyzer unit 22. Such data may include user specific audio data, such as an existing hearing loss, in order to individually configure the audio signal processing unit 24 for optimizing performance. The system further comprises a battery which is not shown in Fig. 1.

In order to protect the user's hearing from excessive sound levels while still enabling a selected perception of ambient sound, for example speech signals, the audio signal processing unit is controlled in such a manner that the levels of the processed audio output signals resulting from input audio signals from noise directions determined by the analyzer unit 22 are reduced relative to the processed audio output signals resulting from input output signals from angular directions other than the determined noise directions in order to comply with a predefined output sound level condition. Thereby undesired noise from noise directions is selectively suppressed relative to desired sound from other directions, i.e. the spatial amplification profile is permanently adapted to the actual spatial noise/sound distribution in order to eliminate disturbing and potentially harmful noise sources.

Preferably, the levels of processed audio output signals resulting from input audio signals from the determined noise directions are reduced such that the total resulting amplification, i.e. the amplification including the acoustic attenuation provided by the earplugs 10, 12, of input audio signals from the noise direction is negative, more preferably less than -10 dB, and most preferably less than -15 dB.

The levels of processed output audio signals resulting from input audio signals from the noise directions may be reduced such that the amplification of input audio signals from the noise directions is frequency selective.

Preferably, also the total resulting amplification, i.e. the amplification including the acoustic attenuation provided by the earplugs 10, 12, of processed audio output signals resulting from input audio signals from non-noise directions is negative.

The amplification of processed audio output signals resulting from input audio signals from non-noise directions may be frequency selective, with speech frequencies being preferably amplified.

Preferably, the sound output level condition depends on frequency, preferably relating to frequency bands. The output sound level condition may be individually selected for the specific user.

According to one embodiment, a plurality of different predefined input sound level conditions and a plurality of associated predefined output sound level conditions are provided, wherein the noise directions are classified depending on which of the predefined input sound level conditions is fulfilled for the respective noise direction, and wherein the levels of processed audio output signals resulting from input audio signals from each of the classified noise directions are reduced depending on the associated predefined output sound level condition.

Preferably, the input sound level condition depends on frequency and, in particular, may relate to frequency bands. In this case, the input sound level condition may include for each frequency band a level limit, wherein an angular direction is judged to be a noise direction if at least one of these level limits is exceeded.

In Figs. 2 to 4 typical noise exposure situations are schematically illustrated. For the sake of simplification it is assumed that all noise sources are located within a plane 20 which is normal to the user's longitudinal body axis. According to Fig. 2, the user wearing the hearing protection system is exposed to moderate homogeneous noise with levels which do not significantly depend on the angular direction in the plane 20. In this case, no noise direction is detected, so that no direction-selective amplification by the audio signal processing unit 24 is necessary. In other words, in the situation shown in Fig. 2 the amplification of ambient sound does not depend on the angular direction. If the noise level in Fig. 2 would be homogeneously increased above a given limit implemented by the input sound level condition in the analyzer unit 22, all angular directions of the plane 20 would be judged to be noise directions, so that in all angular directions the amplification provided by the audio signal processing unit 24 would be reduced accordingly.

In Fig. 3 a common situation is illustrated in which there is at least one pronounced and disturbing noise source 30 which superposes a more or less homogeneous moderate background noise. In this case, the analyzer unit 22 will judge that the angular direction in which the noise source 30 is located is the noise direction which has to be eliminated by corresponding control of the audio signal processing unit 24. Consequently, the audio signal processing unit 24 will be controlled such that input audio signals resulting from a sector 32 in which the noise source 30 is located will be suppressed, at least to some extent, compared to input audio signals from the other angular directions. In practice, in the case of beam forming with two microphones 16, 18 this would mean that amplification of input audio signals from cone envelopes having angular apertures from ϕ₁ to ϕ₂ (this range corresponds to the size of the noise source and the accuracy of the detection) would be reduced.

In Fig. 4 another common situation is illustrated in which multiple strong noise sources 34, 36, 38 and 40 are present which produce disturbing noises having different frequency shapes. In this case it is particularly beneficial if the analyzer unit 22 is provided with a plurality of different predefined input sound level conditions depending on frequency, in particular relating to frequency bands, so that the different noise directions may be classified in order to enable the audio signal processing unit 24 to individually react on the detected noise sources depending on the type of noise produced by the respective noise source. Thereby it is enabled to suppress in the corresponding noise direction, i.e. a corresponding sector 42, 44, 46 and 48, respectively, only those frequencies which have been recognized as noise by the analyzer unit 22, i.e. the audio input signals in the sectors 42, 44, 46 and 48 are not completely suppressed but only in those frequency bands, in which a tolerable threshold is exceeded. Of course, such frequency dependant noise detection/suppression is beneficial also for the case in which only a single noise source is present.

In general, the hearing protection system may be designed as a binaural system (see elements shown in dashed lines in Fig. 1). In this case, each earplug 10, 12 may be provided with a binaural communication unit 50 which communicates bidirectionally with its counterpart in the other earplug via a communication link 52 which may be integrated into means for mechanically connecting the earplugs 10, 12, such as a cord 54, for preventing loss of the earplugs 10, 12. In addition, each binaural communication unit 50 communicates with the respective analyzer unit 22 and the respective audio signal processing unit 24.

In such a system the analyzer unit 22 of the right earplug 10 and the left earplug 12 are enabled to communicate with each other in order to improve the accuracy of the detection of the noise directions.

In a system as described above with two microphones 16, 18 the noise source search results would be averaged over the corresponding cone envelope, which results in a reduced amplification over the entire cone envelope, although the noise source usually will cover only a rather small part of the cone envelope. Hence, in order to improve the selectivity of the hearing protection system, the system may be designed as a system capable of searching angular directions in three-dimensional space for noise directions in such a manner that each angular direction corresponds to a beam in three-dimensional space having a three-dimensional orientation assigned to the respective angular direction. In other words, with such a system the angular resolution is enhanced from a cone envelope to a single beam. In this case, each earplug 10, 12 would be provided with at least three spaced apart microphones. A corresponding method for full three-dimensional localization of sound sources, which would be useful for the present invention, is described, for example, in WO 00/68703.

With such a system it will be possible to more precisely locate noise sources so that in this case only the amplification in a single beam direction would be reduced, and not the amplification over an entire cone envelope.

## Claims

1. A method for providing hearing protection to a person, comprising:
providing each of the person's ears with a hearing protection device (10, 12) in such a manner that an acoustic attenuation of at least 10 dB averaged over the audible frequency range is achieved;
converting, by said hearing protection devices, ambient sound into input audio signals;
assigning, by analyzing phases of said input audio signals, angular directions to said input signals;
permanently determining noise directions (32, 42, 44, 46, 48) by searching, by analyzing levels of said input audio signals, over all angular directions around said person for angular directions having a noise source (30, 34, 36, 38, 40) from which noise impinges on said hearing protection devices, wherein an angular direction is judged to be a noise direction if input audio signals from that direction fulfil a predefined input sound level condition;
processing, in order to produce processed audio signals, said input audio signals in such a manner that the levels of processed audio signals resulting from input audio signals from said noise direction(s) are reduced, relative to processed audio signals resulting from input audio signals from other angular directions, in order to comply with a predefined output sound level condition; and
converting said processed audio signals into sound for being received by said person's ears.

2. The method of claim 1, wherein said levels of processed audio signals resulting from input audio signals from said noise direction(s) (32, 42, 44, 46, 48) are reduced such that the total resulting amplification, including said acoustic attenuation, of input audio signals from said noise direction(s) is negative.

3. The method of claim 2, wherein said levels of processed audio signals resulting from input audio signals from said noise direction(s) (32, 42, 44, 46, 48) are reduced such that the total resulting amplification, including said acoustic attenuation, of input audio signals from said noise direction(s) is less than -10 dB, preferably less than -15 dB.

4. The method of one of the preceding claims, wherein said levels of processed audio signals resulting from input audio signals from said noise direction(s) (32, 42, 44, 46, 48) are reduced such that the amplification of input audio signals from said noise direction(s) is frequency selective.

5. The method of one of the preceding claims, wherein said processed audio signals resulting from input audio signals from said other angular directions are processed such that the total resulting amplification, including said acoustic attenuation, of said processed audio signals resulting from input audio signals from said other angular directions is negative.

6. The method of one of the preceding claims, wherein said processed audio signals resulting from input audio signals from said other angular directions are processed such that the amplification of said processed audio signals resulting from input audio signals from said other angular directions is frequency selective, with speech frequencies being preferably amplified.

7. The method of one of the preceding claims, wherein a plurality of different predefined input sound level conditions and a plurality of associated predefined output sound level conditions are provided, wherein said noise direction(s) (32, 42, 44, 46, 48) are classified depending on which of said predefined input sound level conditions is fulfilled for the respective noise direction, and wherein said levels of processed audio signals resulting from input audio signals from each of said classified noise directions are reduced depending on the associated predefined output sound level condition.

8. The method of one of the preceding claims, wherein said input sound level condition depends on frequency.

9. The method of claim 8, wherein said input sound level condition relates to frequency bands.

10. The method of claim 9, wherein said input sound level condition includes for each frequency band a level limit, wherein an angular direction is judged to be a noise direction if at least one of said level limits is exceeded.

11. The method of one of the preceding claims, wherein said output sound level condition depends on frequency.

12. The method of claim 11, wherein said output sound level condition relates to frequency bands.

13. The method of one of the preceding claims, wherein said input sound level condition and/or said output sound level condition are individually selected.

14. The method of one of the preceding claims, wherein said converting of ambient sound into said input audio signals is effected by at least two spaced apart microphones (16, 18), and wherein said input audio signals are assigned to said angular directions by determining the respective phase difference between the output signals of said microphones.

15. The method of one of the preceding claims, wherein each of said angular directions corresponds to an envelope of a cone having an angular aperture assigned to said respective angular direction.

16. The method of one of claims 1 to 14, wherein each of said angular directions corresponds to a beam in three-dimensional space having a three-dimensional orientation assigned to said respective angular direction.

17. A hearing protection system comprising
a first hearing protection device (10) to be worn at a person's right outer ear and/or at least partly within the person's right ear canal and a second hearing protection device (12) to be worn at the person's left outer ear and/or at least partly within the person's left ear canal, each of said hearing protection devices being adapted to provide for an acoustic attenuation of at least 10 dB averaged over the audible frequency range when worn by said person, wherein at least two spaced apart acoustic input transducers (16, 18) for converting ambient sound into input audio signals are provided,
an analyzer unit (22) for assigning, by analyzing phases of said input audio signals, angular directions to said input signals and for permanently determining noise directions (32, 42, 44, 46, 48) by searching, by analyzing levels of said input audio signals, over all angular directions around said person for angular directions having a noise source (30, 34, 36, 38, 40) from which noise impinges on said hearing protection devices, wherein an angular direction is judged to be a noise direction by said analyzer unit if input audio signals from that direction fulfil a predefined input sound level condition;
an audio signal processing unit (24) for processing, in order to produce processed audio signals, said input audio signals in such a manner that the levels of processed audio signals resulting from input audio signals from said noise direction(s) are reduced, relative to processed audio signals resulting from input audio signals from other angular directions, in order to comply with a predefined output sound level condition; and
at least one acoustic output transducer (26) for converting said processed audio signals into sound for being received by said person's ears.

18. The hearing protection system of claim 17, wherein each hearing protection device (10, 12) is equipped with at least two spaced apart acoustic input transducers (16, 18).

19. The hearing protection system of one of claims 17 or 18, wherein each hearing protection device (10, 12) is equipped with an acoustic output transducer (26).

20. The hearing protection system of one of claims 17 to 19, wherein said hearing protection devices (10, 12) are connected by a communication link (52).

21. The hearing protection system of claim 20, wherein said communication link (52) is integrated into means (54) for mechanically connecting said hearing protection devices (10, 12).

22. The hearing protection system of one of claims 17 to 21, wherein each hearing protection device is designed as an earplug (10, 12) having a shell (14).

23. The hearing protection system of claim 22, wherein the outer surface of said shell (14) is individually shaped according to the measured inner shape of the user's outer ear and ear canal.

24. The hearing protection system of claim 23, wherein said shell (14) is a hard shell with an elasticity of from shore D85 to shore D65.
